# EUROPEAN PATENT APPLICATION

(11) **EP 2 347 660 A1**
(43) Date of publication of application: **27.07.2011**
(21) Application number: 08877187.8
(22) Date of filing: 03.10.2008
(51) Int. Cl.: A23L 1/29, A23L 1/30, A61P 3/04, A61P 3/06, A61K 31/201, A61K 36/82, A61K 36/77, A23C 9/20, A23L 2/52

(54) **COMPOSITION FOR PROMOTING CONTROL OF TOTAL AND LDL CHOLESTEROL AND/OR WEIGHT LOSS AND/OR THERMOGENESIS**

(71) Applicant: Sigma Alimentos, S.A. De C.V., Neuvo Leon (MX)
(72) Inventor: LAVALLE GONZÁLEZ, Fernando Javier, Nuevo León (MX); BOSQUES PADILLA, Francisco Javier, Nuevo León (MX); NARANJO MODAD, Sandra, Nuevo León (MX); MORENO CAMPAÑA, Víctor Manuel, Nuevo León (MX); MARÍN REBOLLEDO, Víctor, Nuevo León (MX)
(74) Representative: Elzaburu Marquez, Alberto
(86) International application number: PCT/MX2008/000133
(87) International publication number: WO 2010/039019

(57) **Abstract**

A composition for promoting control of total and LDL cholesterol and/or weight loss and/or thermogenesis in individuals, the composition including green tea extract in a quantity of about 200 mg to about 300 mg; guarana extract in a quantity of about 160 mg to about 260 mg; yerba mate extract in a quantity of about 100 mg to about 300 mg; and conjugated linoleic acid in a quantity of about 700 mg to 3400 mg. Additionally, the green tea extract is standardized in a content of at least 50% epigallocatechin gallate (EGCG), while the conjugated linoleic acid is standardized in a content of at least 50% of trans10, cis 12-CLA (10-12 CLA), and 50% cis9, trans11-CLA (9-11 CLA).

## Description

### TECHNICAL FIELD OF THE INVENTION

The present invention relates to nutritional compositions of natural origin, and particularly to a composition based on natural extracts that promotes control of total cholesterol and low density lipoproteins, weight loss and/or thermogenesis in individuals.

### BACKGROUND OF THE INVENTION

Obesity can be defined as excess body fat which is manifested in increased weight. This increase in fat accumulation is a consequence of excess energy consumption (excess nutrition) and a decrease in the consumption of said nutrition (reduced physical activity).

For practical purposes, it is generally accepted that there is overweight if the body weight exceeds the "desirable weight", however, there is obesity if the body weight is 20% or more over the "desirable weight" (Council on Scientifc Affairs, 1988, Treatment of Obesity in Adults. JAMA 260, 2547-48). The desirable weight of a human being can be defined according the Metropolitan Height and Weight Tables (Council on Scientifc Affairs, 1988, Treatment of Obesity in Adults. JAMA 260, 2547-48) as the midpoint of the range for individuals of medium build.

Obesity can be classified by the percentage of fat, as mild (20-30%), moderate (30-60%) or severe (greater than or equal to 60%), or in terms of Body Mass Index (BMI) that is calculated dividing the weight in kilograms of the individual between the height in meters to the square root, with a result below 18.5 is considered low weight, from 18.5 to 25 is considered the appropriate weight, and from 25 to 30 is considered overweight and over 30 is obese.

Obesity is accompanied by a number of health hazards. This may adversely affect both heart and lung functions, disrupt endocrine functions and may cause emotional problems, and even give rise to the so-called metabolic syndrome, all together a set of risk factors of metabolic origin that increase the risk of diabetes, coronary artery disease, apoplexy and peripheral vascular disease, and in general it is characterized by abdominal obesity, dyslipidemia (elevated triglycerides), high blood pressure, intolerance to the glucose and hypercoagulation (tendency to form excess blood clots).

Hypertension, glucose intolerance and diabetes mellitus insulin non-dependent and hypercholesterolemia are more common states in overweight individuals than in normal weight individuals. Obesity can contribute to both the morbidity and mortality of individuals who suffer, for example, from hypertension, infarction, type II diabetes mellitus, some cancers, gallbladder disease and ischemic heart disease. It is known that moderate and severe obesity increase mortality. Colon cancer and rectal cancer are diseases which frequently occur in obese men, and obese women frequently suffer from cancer of the uterus or gall bladder. It has been observed that an increase in overweight has almost as a consequence increased psychological and social problems.

The causes of obesity are many and complex and how they relate to each other is not completely known. Obesity may be the result of lifestyle, that is, physical inactivity and indiscriminate consumption of food and/or a result of genetic susceptibility of obese individuals. Genetic influences are generally considered an important role in determining the fat and obesity in humans.

The basic principle of treatment of obesity or overweight is to establish a negative energy balance. A negative energy balance can be obtained mainly by using three different methods of treatment or their combination.

First, an effective treatment is to reduce the intake or ingestion of energy, that is, the ingestion of food. This is essentially only possible through a dietetic treatment and in some cases by some medications. The dietetic treatment should consist of a weight reduction diet and a maintenance diet. After successful weight loss, energy supply should be increased slowly until the weight stabilizes at a food supply that is nutritious and acceptable to the patient. The importance of long-term diet is estimated by the fact that only 10 to 20% of the patients are able to maintain the reduced weight they have reached.

Second, the increase in physical activity will lead to an increased energy waste and, therefore, contributes to a negative energy balance. However, to obtain a significant weight loss, hours of daily exercise will be required. Physical activity alone therefore plays a reduced role in the treatment of obesity, although it is a very important supplement to other types of treatment. Also, physical activity can contribute to energy expenditure after a dietary treatment comprising an energy restriction.

Third, drugs can be used in the treatment of obesity alone or in combination with a diet and/or increased physical activity. The drugs used in the treatment of obesity may be drugs that reduce appetite and/or thermogenic drugs. However, frequently, there is some overlap within these two categories. Drugs that reduce appetite exert their effect primarily by decreasing energy intake or ingestion of energy. The reduction of food consumption is a consequence of the action of the drug in the brain transmitter systems that are involved in regulating the appetite. The action of these drugs is supposed to receive the intermediary action of the hypothalamus in several places. The action may be carried out through the adrenergic dopaminergic and serotoninergic pathway, or a combination thereof. Whatever the system, the end result is a stimulation of the satiety center and eventually a simultaneous decrease of the feeding activity, which results in a reduced appetite. Examples of agents known as appetite-reducing, for example, ephedrine, phenylpropanolamine, amphetamines and fenfluramine. It should be noted that mixtures based on ephedrine are not approved as a treatment for control of overweight and obesity due to their adverse health effects (hypertension and risk of myocardial infarction).

Thermogenic drugs for the treatment of obesity is now generally accepted, as they have potential therapeutic value, and in recent years there has been a growing interest in finding new thermogenic compounds. The concern relates primarily to the suggestion well accepted that obesity can be determined genetically. The genetic defect responsible for the possible development of obesity relates to a thermogenic defect (ie a defect in the metabolic system) of obese people (Dulloo, A. and D.S. Miller, 1989, Ephedrine, caffeine and aspirin: "Over-the-counter" drugs that interact to stimulate thermogenesis in the obese. Nutrition 5, 7-9). If the nature of the thermogenic defect is completely clear, there is a notable evidence that indicates a defective reactivity of the sympathoadrenal system (Astrup, A.V., 1989, Treatment of obesity with thermogenic agents, Nutrition 5, p. 70). Dullo and Miller suggest that the thermogenic defect on obese persons refer to the reduced liberation of norepinephrine, more than to the insensibility to Neurotransmitter. The drugs that imitate the activity of the sympathetic nervous system and increase the metabolic rate offer therefore considerable therapeutic potential for the treatment of obesity.

A thermogenic drug can be defined as a drug capable of increasing metabolic rate, ie. increasing energy expenditure. Among the known thermogenic drugs can be mentioned, for example, ephedrine, epinephrine, norepinephrine (Astrup A., 1986, Thermogenesis in human brown adipose tissue and skeletal muscle induced by sympatomimetic stimulation. Acta Endocrinol. 112, suppl. 278, 1-32), isoproterenol, phenylpropanolamine and caffeine (Hollands, M.A., J.R.S. Arch and M.A. Cowthorne, 1981, A simple apparatus for comparative measurements of energy expenditure in human subjects: The thermic effect of caffeine. Am.J.Clin.Nutr. 34, 2291-2294).

Human obesity may also be treated with Orlistat® (see U.S. patent US-4,598,089), whose brand name is Xenical® of Roche®, also known as tetrahydrolipostatin. Its main function is preventing the absorption of fats in the human diet, thus reducing caloric intake. Orlistat® works by inhibiting pancreatic lipase, an enzyme that breaks down triglycerides in the intestine. Without this enzyme, triglycerides from the diet are not hydrolyzed into absorbable fatty acids and are excreted undigested. Only a small quantity of Orlistat® is absorbed systemically; its main effect is local lipase inhibition within the gastrointestinal tract after consumption of an oral dose. The main means of elimination is via feces.

Currently you can also find thermogenic supplements or compositions based on combinations of different herbs and extracts that act efficiently. Examples of current thermogenic compositions are described in the following patent documents:

Ira Jacobs, Paul T. Gardiner and Michael Molina, in the publication of the U. S. patent application US-2005/0130933 A1, describe a supplement for the loss of weight that comprises an ingredient that promotes the satiety, which includes a mixture of dietetic fibres, including glucomanan, a gum and a thermogenic ingredient selected from a group consisting of caffeine, catechin polyphenol, and combinations thereof.

William Stewart Brown, Tara Nadine Stubensey, Alan Christopher Logan and Alicja Wojewnik Smith, in the publication of U.S. patent application US-2004/0202732 A1, describes a synergistic composition and preferably green tea extract from, for example, epigallocatechin gallate (EGCG) and linoleic acid joined together to assist the loss of weight in humans.

Inna Yegorova, in the publication of U.S. patent application US-2003/0082168 A1, describes a composition for facilitating weight loss, inhibit the absorption of carbohydrates, enhance lipolysis and modulate glucose metabolism in humans. The composition comprises wheat alpha-amylase, conjugated linoleic acid, lipotropic vitamins and green tea.

Marcin Krotkiewski, in the publication of the international patent application WO-2005/067952 A1, describes a formulation for treatment of obesity and the associated metabolic syndrome; the formulation combines a green tea extract containing epigallocatechin gallate (EGCG), *Coleus forskholii* extract, *Betula alba* extract, and guarana extract or green tea extract.

Max Rombi, in the publication of the Mexican patent application MX-P01007191 A, discloses a composition for curative and prophylactic treatment of obesity, including a green tea extract rich in catechins, in particular containing from 20% to 50% by volume of catechins expressed in epigallocatechin gallate (EGCG).

José A. Diaz and Eduardo M. Naranjo, in the U. S. patent US-6,932,987 B1, describe a chemical composition in the form of tablets that improves the metabolism, the composition is formed by approximately 100 mg of L-carnitine, approximately 50 mg of conjugated linoleic acid, a predetermined quantity of ginger extract, approximately 50 mg of green tea extract, approximately 50 mg of *Citrus aurantium,* and approximately 25 mg of capsaicin.

Wayne F. Gorsek, in the U. S. patent US-6,565,847 B1, describes a formulation for the loss of weight that contains green tea extract, hydroxycitric acid, thermogenic herbs, glucomanane, chrome and a probiotic. The formulation increases the basic metabolism, suppresses the appetite and helps to use fat without provoking adverse cardiovascular effects.

As can be observed, the compositions described above are applicable only in weight reduction and increased thermogenesis, but do not carefully consider a control of the content of caffeine in them, according to applicable health requirements, neither to other health indicators that accompany obesity and play an important role in certain pathologic states in the human. Examples of these other indicators could be the alteration of cholesterol metabolism, balance of the contents of lipoproteins responsible for cholesterol transport, alteration of blood pressure, etc.

As it is known, cholesterol deposits in the vascular system have been known to cause various pathologic disease states, including coronary heart disease.

Humans get cholesterol in two ways, in an exogenous way, directly through food and endogenously through the synthesis in the body cells from acetyl-CoA.

Cholesterol biosynthesis occurs in the endoplasmic reticulum (smooth) of virtually all animal cells. The main steps of the synthesis of cholesterol are: Acetyl-CoA is converted to mevalonate; mevalonate is converted into squalene by successive transfer reactions of prenyl groups; squalene is converted into lanosterol and lanosterol is converted to cholesterol after another 21 successive reactions, enzymatically catalyzed.

It is now known that cholesterol is transported in the blood in the form of complex particles consisting of a core of cholesterol esters, plus triglycerides, and an outer part consisting mainly of phospholipids and various types of protein that are recognized by specific receptors. For example, cholesterol is transported to the points of deposit in blood vessels in the form of low-density lipoprotein cholesterol (LDL) and retires from such points of deposit in the form of high density lipoprotein cholesterol (HDL).

Now, the causal role is widely recognized of LDL cholesterol in the pathological state of atherosclerosis. Thus, the sustained presence of elevated levels of LDL cholesterol above recommended levels, increases the risk of cardiovascular events (mainly acute myocardial infarction). Interestingly, HDL cholesterol exerts a protective role of the cardiovascular system. Thus, cholesterol has a dual and complex impact on the physiopathology of atherosclerosis, and therefore cardiovascular risk estimation based only on total plasma cholesterol levels is clearly insufficient.

However, considering the above, clinically defined levels of total plasma cholesterol (the quantity of cholesterol present in all lipoprotein classes) recommended by the American Society of Cardiology are:
- Cholesterolemia below 200 mg/dL (milligrams per deciliter): the concentration desirable for the general population, usually correlating with a low risk of cardiovascular disease.
- Cholesterolemia between 200 and 239 mg/dL: an intermediate risk in the general population, but elevated in people with other risk factors like diabetes mellitus.
- Cholesterolemia above 240 mg/dL: can determine a high cardiovascular risk and recommended to initiate a change in lifestyle, especially with regard to diet and physical exercise.

Strictly speaking, the desirable level of LDL cholesterol is clinically to be defined for each individual based on their individual cardiovascular risk, which is determined by the presence of various risk factors, among them: age and sex, family history, smoking, presence of hypertension, HDL cholesterol level.

In people with high cardiovascular risk, that is, those with a probability of more than 20% of suffering a major cardiovascular or lethal event over a period of 10 years, such as diabetic patients or those who have previously had one of these events. Currently the recommendation is to keep their LDL cholesterol below 100 mg/dL. Even in patients who are rated as very high risk an LDL cholesterol is recommended equal to or below 70 mg/dL.

After these discoveries, the search for therapeutic agents that control blood cholesterol has shifted to the discovery of compounds that were more selective in their action, that is, agents that are effective in raising blood serum levels of HDL cholesterol and/or decrease the LDL cholesterol levels. While these agents are effective in moderating the levels of serum cholesterol, they have little or no effect on controlling the initial absorption of dietary cholesterol in the body through the bowel wall.

In the intestinal mucosa cells, cholesterol ingested in the diet is absorbed as free cholesterol which must be esterificated by the action of the enzyme acyl-CoA: cholesterol acyltransferase (ACAT) before it can be packaged in chylomicrons that are subsequently released into the bloodstream. Thus, therapeutic agents which effectively inhibit the action of ACAT prevent the intestinal absorption of dietary cholesterol in the blood stream or the reabsorption of cholesterol which was released earlier in the intestine through the body's own regulatory action.

In view of this, it is necessary to provide a composition based on natural plant extracts in a synergistic manner and control of caffeine promote control of total cholesterol and LDL cholesterol, and that in turn is a thermogenic composition that promotes weight reduction and thermogenesis in individuals. Just as it is necessary to provide food products with said composition.

### SUMMARY OF THE INVENTION

In view of the above mentioned and the purpose of providing solutions to the constraints encountered, it is the subject of the invention to provide a composition for promoting control of total cholesterol and low density lipoprotein (LDL) in individuals; the composition includes green tea extract in a quantity of about 200 mg to about 300 mg; a guarana extract standardized in its contents of caffeine and in a quantity of about 160 mg to about 260 mg; yerba mate extract standardized in its contents of caffeine and in a quantity of about 100 mg to 300 mg; and conjugated linoleic acid in a quantity of about 700 mg to about 3400 mg.

Another object of the invention is to offer a composition for promoting weight loss in individuals, the composition includes green tea extract in a quantity of about 200 mg to about 300 mg; guarana extract in a quantity of about 160 mg to about 260 mg; yerba mate extract in a quantity of about 100 mg to about 300 mg; and conjugated linoleic acid in a quantity of about 700 mg to about 3400 mg.

Another object of the invention is to offer a composition for promoting thermogenesis in individuals, the composition includes green tea extract in a quantity of about 200 mg to about 300 mg; guarana extract in a quantity of about 160 mg to about 260 mg; yerba mate extract in a quantity of about 100 mg to about 300 mg; and conjugated linoleic acid in a quantity of about 700 mg to about 3400 mg.

Another object of the invention is the use of a composition that comprises green tea extract in a quantity of about 200 mg to about 300 mg; guarana extract in a quantity of about 160 mg to about 260 mg; yerba mate extract in a quantity of about 100 mg to about 300 mg; and conjugated linoleic acid in a quantity of about 700 mg to about 3400 mg, to prepare a food product to promote weight loss, control of low-density lipoprotein cholesterol (LDL cholesterol) and/or thermogenesis in individuals.

Additionally, for each of the objects of the invention the green tea extract is standardized to a content of at least 50% of epigallocatechin gallate (EGCG), while the conjugated linoleic acid is standardized to a content of at least 50% of trans10, cis 12-CLA (10-12 CLA) and a content of at least 50% of cis9, trans11-CLA (9-11 CLA).

Also, for each of the objects of the invention, the green tea extract contributes a quantity of about 10 mg to about 15 mg of caffeine, the guarana extract contributes a quantity of about 35 mg to about 58 mg of caffeine, and yerba mate extract contributes a quantity of about 9 mg to about 27 mg of caffeine.

### BRIEF DESCRIPTION OF THE FIGURES

The characteristic details of the present invention are described in the following paragraphs, together with the figures related to it, in order to define the invention, but not limiting the scope of it.
Figure 1 illustrates a diagram of total weight loss after 12 weeks of follow-up of groups of individuals, subjects of the study in accordance with an embodiment of the invention.
Figure 2 illustrates a diagram of total weight loss after 12 weeks of follow-up of groups of individuals, subjects of the study, who consumed yoghurt supplemented in accordance with an embodiment of the invention.
Figure 3 illustrates a diagram of total weight loss from Figure 1 explained in a corporal composition, after 12 weeks of follow-up of groups of individuals, subjects of the study in accordance with an embodiment of the invention.
Figure 4 illustrates a diagram of low-density lipoprotein cholesterol (LDL cholesterol) after 12 weeks of follow-up of groups of individuals, subjects of the study, who consumed yoghurt supplemented in accordance with an embodiment of the invention.
Figure 5 illustrates a diagram of total cholesterol loss after 12 weeks of follow-up of groups of individuals, subjects of the study, who consumed yoghurt supplemented in accordance with an embodiment of the invention.

### DETAILED DESCRIPTION OF THE INVENTION

The characteristic details of this invention are described in the following paragraphs, which have the objective of defining the invention, but without limiting its scope.

The composition for promoting control of total cholesterol and low density lipoprotein, weight loss and/or thermogenesis in individuals according to the invention shows components which in turn may consist of multiple components.

The components are described individually below, without necessarily being described in any order of importance.

### GREEN TEA EXTRACT

This is the traditional tea plant known as *Camellia Sinensis.* It contains many phytoactive substances, but those who have a greater effect in reducing fat are the polyphenols especially one known as EGCG. Also contains a quantity of caffeine but much less than coffee. Green tea is also rich in important minerals such as selenium or manganese.

Studies on the action of green tea include those who worked on the study of the mechanisms by which catechins can help to reduce serum lipids. The most active catechin appears to be EGCG, which has a marked antioxidant effect, which apparently may be greater than that of vitamin C and E, plus giving thermogenic properties and increased use of fat for energy. Among its benefits stands out the modulation of LDL (CHRISTINA A. BURSILL and PAUL D. ROACH, Modulation of Cholesterol Metabolism by the Green Tea Polyphenol (-)-Epigallocatechin Gallate in Cultured Human Liver (HepG2) Cells, J. Agric. Food Chem. 2006, 54, 1621-1626, and Sung I. Koo and Sang K. Noh, "Green tea as inhibitor of the intestinal absorption of lipids: potential mechanism for its lipid-lowering effect", Journal of Nutritional Biochemistry 18 (2007) 179-183).

Green tea catechins and therefore contained therein, increase the utilization of fat as fuel but unlike other thermogenics do not accelerate the heart rate.

### GUARANA EXTRACT

Guarana extract is derived from a plant called *Paullinia Cupana,* which comes from the Brazilian Amazon. Contains caffeine, theobromine, tannins, catechins, saponins and colline, apart from essence oils.

Guarana extract has a tonic effect, an ergogenic effect (basically by its caffeine) and a lipolytic effect. All these effects are given by the properties that has caffeine, theobromine, and tannins that are contained in it in a natural way. It behaves like a nervous system stimulant, especially sympathoadrenal system, which results in an increased basal metabolism, oxidation of fats and wakefulness.

Scientific studies in humans support the benefits of caffeine to reduce fat (Boozer CN, Nasser JA, Heymsfield SB, et al. An herbal supplement containing ma huang-guarana for weight loss: a randomized, double-blind trial. Int J Obes Relat Metab Disord, 2001, 25:316-324*)* and as an ergogenic aid for increased basal metabolism.

### YERBA MATE EXTRACT

Yerba mate contains antioxidant polyphenols similar to those of the tea. Yerba mate may increase fat metabolism (Martinet A, Hostettmann K, Schutz Y, et al. Thermogenic effects of commercially available plant preparations aimed at treating human obesity. Phytomedicine, 2000, 6:231-8*.),* and on this basis, yerba mate has been proposed as an agent to lose weight.

### CONJUGATED LINOLEIC ACID

Conjugated linoleic acid (CLA) is the generic name for a group of conjugated isomers of linoleic acid (polyunsaturated fatty acid of the omega-6 series) and is found naturally in vegetable oils and in a greater proportion of food of animal origin, such as ruminant meat and milk products. The two main isomers of CLA are the trans10, cis 12-CLA (10-12 CLA) and cis9, trans11-CLA (9-11 CLA).

Conjugated linoleic acid in the body acts on two levels: It inhibits the transport of triglycerides from the blood to the adipocytes, reducing lipogenesis (fat tissue formation) and facilitates fat burning generating energy in the mitochondria (favors lipolysis).

Although often called a supplement for weight loss, preliminary evidence suggests that CLA may help you lose fat while retaining muscle (Blankson H, Stakkestad JA, Fagertun H, et al. Conjugated linoleic acid reduces body fat mass in overweight and obese humans. J Nutr. 2000;130:2943 - 2948; Smedman A, Vessby B. Conjugated linoleic acid supplementation in humans - metabolic effects. Lipids. 2001;36:773 - 781 and Thom E. et al. Conjugated linoleic acid reduces body fat in healthy exercising humans. J Int Med Res 2001, 29:392-396). The total effect could be an improvement in the body composition (proportion of muscle to fat) instead of weight loss.

A clinical double-blind trial found evidence that CLA may be useful for high cholesterol (Noone EJ, Noone EJ, Roche HM, et al. The effect of dietary supplementation using isomeric blends of conjugated linoleic acid on lipid metabolism in healthy human subjects. BrJ Nutr. 2002;88:243-251).

### MIXTURE

The composition for promoting control of total cholesterol and low density lipoprotein, weight loss and/or thermogenesis in individuals according to the invention shows components described in the following quantities.
a) from about 200 mg to about 300 mg of green tea extract,
b) from about 160 mg to about 260 mg of guarana extract,
c) from about 100 mg to about 300 mg of yerba mate extract, and
d) from about 700 mg to about 3400 mg of conjugated linoleic acid.

The additional composition for promoting the control of total cholesterol and low density lipoprotein, weight loss and/or thermogenesis in individuals according to the invention contains the following:
e) an ingredient to satiate the appetite, for example, glucomannan gum.

Glucomannan is a polysaccharide (complex carbohydrate) nondigestible extracted from tubercle of an Asian plant called *Amorphophallus konjac.* Glucomannan can absorb over 100 times its volume in water to form a thick gel. Ingested along with plenty of fluids it has a satiating effect that reduces the sense of "empty stomach" and thus reduces the appetite or the need to eat. Furthermore, experimental and clinical studies have shown that it helps to reduce LDL cholesterol levels.

Other gums which may be employed in the composition are xanthan gum and guar gum.

The total caffeine content of the composition to promote weight loss, control of low density lipoprotein and/or thermogenesis in individuals according to this invention is within the applicable health standards and is defined by the accumulation of individual contribution of caffeine of each of the components described above:
- the contribution of caffeine of the green tea extract is within the range of about 10 mg to about 15 mg of caffeine,
- the contribution of caffeine of the guarana extract is within the range of about 35 mg to about 58 mg, and
- the contribution of caffeine of the yerba mate extract is within the range of about 100 mg to about 300 mg of the extract of yerba mate.

In order to supply food products that allow for promoting control of total cholesterol and low density lipoprotein, weight loss and/or thermogenesis in individuals according to this invention, it can be added to milk products, juices or drinks, before or after their pasteurization. The stability of the composition and of its active ingredients, for example, catechins and, particularly, of EGCG's, will depend on the storage form of the product and its packing. Preferably, it must be packed in a package that does not allow any light, oxygen come through, and use a food composition with an acid pH to avoid degradation of the active ingredients. The ideal food base composition for use of the composition are milk products, juices and drinks packaged in cans with a pH lower than 4.5 and preferably less than 3.

Additionally, the composition of the invention may be in the form of a tablet, capsule or as a nutritional supplement.

### EXAMPLES OF EMBODIMENTS OF THE INVENTION

The invention will now be described with respect to the following examples, which are solely for the purpose of representing the way of carrying out the implementation of the principles of the invention. The following examples are not intended as a comprehensive representation of the invention, nor try to limit the scope thereof.

Table 1 shows examples of compositions for promoting control of total cholesterol and low density lipoprotein, weight loss and/or thermogenesis in individuals according to the invention. Said table illustrates the contribution of caffeine in each of the components.

| Example | Green tea extract content (mg) | Guarana extract content (mg) | Yerba mate extract content (mg) | Conjugated linoleic acid content | Total caffeine (mg) |
|---|---|---|---|---|---|
| | Individual contribution of caffeine (mg) | Individual contribution of caffeine (mg) | Individual contribution of caffeine (mg) | Individual contribution of caffeine (mg) | |
| 1 | 200 | 260 | 100 | 700-3400 mg | - |
| | 10 | 57.20 | 9 | 0 | 76.2 |
| 2 | 210 | 250 | 120 | 700-3400 mg | - |
| | 10.50 | 55 | 10.80 | 0 | 76.3 |
| 3 | 220 | 240 | 140 | 700-3400 mg | - |
| | 11 | 52.80 | 12.60 | 0 | 76.4 |
| 4 | 230 | 230 | 160 | 700-3400 mg | - |
| | 11.50 | 50.60 | 14.40 | 0 | 76.5 |
| 5 | 240 | 220 | 180 | 700-3400 mg | - |
| | 12 | 48.40 | 16.20 | 0 | 76.6 |
| 6 | 250 | 210 | 200 | 700-3400 mg | - |
| | 12.50 | 46.20 | 18 | 0 | 76.7 |
| 7 | 260 | 200 | 220 | 700-3400 mg | - |
| | 13 | 44 | 19.80 | 0 | 76.8 |
| 8 | 270 | 190 | 240 | 700-3400 mg | - |
| | 13.50 | 41.80 | 21.60 | 0 | 76.9 |
| 9 | 280 | 180 | 260 | 700-3400 mg | - |
| | 14 | 39.60 | 23.40 | 0 | 77 |
| 10 | 290 | 170 | 280 | 700-3400 mg | - |
| | 14.50 | 37.40 | 25.20 | 0 | 77.1 |
| 11 | 300 | 160 | 300 | 700-3400 mg | - |
| | 15 | 35.20 | 27 | 0 | 77.2 |
| Additionally, the EGCG content of the above compositions of the example is approximately at least 80 mg | | | | | |

### Example 1

A single-blind, controlled trial of the effects on weight, BMI and body composition, control of total cholesterol and LDL, thermogenesis and safety in patients with obesity in the administration of yogurt supplemented with the composition of the invention over a period of 12 weeks, at a variable dosage, compared with a placebo.

The study was conducted at Hospital Universitario of Universidad Autonoma de Nuevo Leon in Monterrey, Nuevo Leon, Mexico.

### Objectives

Evaluate the effects on weight, BMI and body composition, control of total cholesterol and LDL, thermogenesis and safety in patients with obesity in the administration of yogurt supplemented with the composition of the invention over a period of 12 weeks, at a low dosage (one yoghurt a day).

Evaluate the effects on weight, BMI and body composition, control of total cholesterol and LDL, thermogenesis and safety in patients with obesity in the administration of yogurt supplemented with the composition of the invention over a period of 12 weeks, at a high dosage (two yoghurts a day).

### Study Design

The study was designed as a cohort study, experimental, comparative, longitudinal, prospective, blind, and placebo-controlled.

Three groups were formed from 60 patients according to Table 2.

**Table 2**

| Group | Member patients | |
|---|---|---|
| Control Group I | 10 obese patients with type I | 10 obese patients with type II |
| Low Dose Group II | 10 obese patients with type I | 10 obese patients with type II |
| High Dose Group III | 10 obese patients with type I | 10 obese patients with type II |

### Patient Selection

We included 60 patients aged 18 to 60 years who had given their consent after being informed, of whom 30 patients had type I obesity (BMI between 27 and 29.9) and 30 patients had type II obesity (BMI between 30 and 32.5) according to the Mexican official standard (Norma Oficial Mexicana NOM) for the treatment and diagnosis of obesity.

In case of women of potential pregnancy, ensuring that they were using safe contraceptive method.

Excluded:
■ Patients with arterial hypertension and/or receiving antihypertensive treatment different from the diuretic,
■ pregnant or lactating women or women wishing to become pregnant,
■ patients with different diseases that could interact with the handling of evidence or in cases where the test treatment could be a potential risk to the health of the patient, for example, patients with gastrointestinal diseases that could delay the absorption of the treatment; heart diseases such as arrhythmia, WPW syndrome, and diseases by cardiac decomposition; severe endocrinological diseases that require treatment (diabetes type I or type II); or diseases of the thyroid gland,

■ patients with malignant diseases, e.g. cancer,
■ patients with psychosis or drug addiction,
■ patients who days before the test were treated with drugs that are known to generate overweight,
■ patients who had undergone surgery to treat overweight,
■ patients with abnormal laboratory results at the time of entry, which could indicate that their participation could be harmful to their health, and
■ patients who started treatment to lose weight.

However, the increased levels of serum triglyceride and serum cholesterol did not exclude patients.

50 patients completed the study, 17 of Control Group I, 17 from Low Dose Group II and 16 from High Dose Group III. In all cases it was recorded if withdrawals or dropouts were due to treatment failure, adverse reactions to treatment, abnormal laboratory values, complications of the disease, pregnancy, non-compliance, unwillingness to participate in the test or other; it was observed that the 10 patients who did not finish the study did so because they did not wish to continue participating.

### Given Treatment

Control Group I: one dose in the morning and one in the evening of 250 ml natural yogurt (placebo) each.

Low Dose Group II: a dose of 250 ml natural yogurt in the morning and one dose at night, 250 ml natural yoghurt added with a mixture of 200 mg of green tea extract standardized to 50% content of EGCG and an additional caffeine of 10 mg; 260 mg guarana extract with a additional 57.20 mg of caffeine; 100 mg yerba mate extract with a additional 9 mg of caffeine, and 1000 mg of conjugated linoleic acid.

High Dose Group III: a dose of 250 ml natural yogurt in the morning and one at night, added with a mixture of 200 mg of green tea extract standardized to 50% content of EGCG and an additional 10 mg of caffeine; 260 mg guarana extract with a additional 57.20 mg of caffeine; 100 mg yerba mate extract with a additional 9 mg of caffeine, and 1000 mg of conjugated linoleic acid.

During the study, no other treatment for overweight or obesity was allowed. Allowed only were treatments that interacted with the treatment that was the object of this study.

### Study Plan

Before starting the study, patients were informed with respect thereto, the consideration of their initial clinical status and voluntary consent was obtained to participate in the study after giving this information.

Age, height, weight and sex of patients were recorded with their concurrent medications and caffeine consumption and the patients received dietary instructions. The nutritional recommendation was fixed for a set diet at 1500 Kcal.

On the first visit, patients were randomly assigned to each group, according to the former Table 2, with the same ratio for men and women.

Every four weeks for a period of 12 weeks, patients were examined by a doctor, carrying out biochemical measurements and body composition for effects of the objectives of the study, and then conducted a follow-up visit two weeks after the last visit.

### Impact assessment

Thyroid Profile: On one occasion and the beginning of the study for each patient a Quantitative Determination of thyrotropin (TSH), thyroxine (T4), Total Triiodothyronine (T3) and free thyroxine (FT4) in serum and plasma was performed, using immunoassay in vitro of electrochemiluminescence (ECLIA), with Elecsys reactives, used automatic analyzers Roche® Elecsys 1010/2010, with determination of free T3 through the technique of Radio-Immune-Analysis.

Body weight: Patients were weighed on a scale at each visit. A scale was used as part of the equipment Bod Pod® Body Composition Tracking System from Life Measurements Inc. The scale was calibrated every week.

Basal energy expenditure: Patients were tested for indirect calorimetry at each visit. The equipment CardiCoach® from Korr Medical Instruments was used.

Body composition: Measuring of the Fat Free Body Mass (FFBM) and Fat Mass (FM), understood the first as the integrated mass of all body components except for the fat, and the second as the quantity of fat stored in the body as an energy reserve, of each of the patients related to the body composition was performed during the 12 weeks through the use of the equipment Bod Pod® Body Composition Tracking System from Life Measurements Inc, in addition the quantity of Localized Corporal Fat Mass, the Fat Free Mass, the total water, and the estimated Muscular Mass, based on the data obtained through the Dual-emission X-ray absorptiometry (DXA), and using the Bioelectrical impedance analysis (BIA), all this, through the equipment Tanita®.

Control of total cholesterol and LDL cholesterol: The treatment effect on total cholesterol and LDL cholesterol in patients was carried out through blood tests in serum and plasma.

### Results obtained

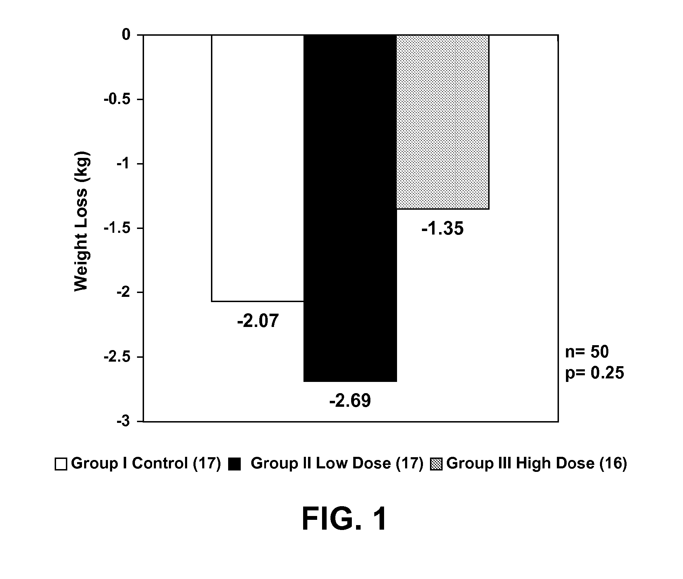

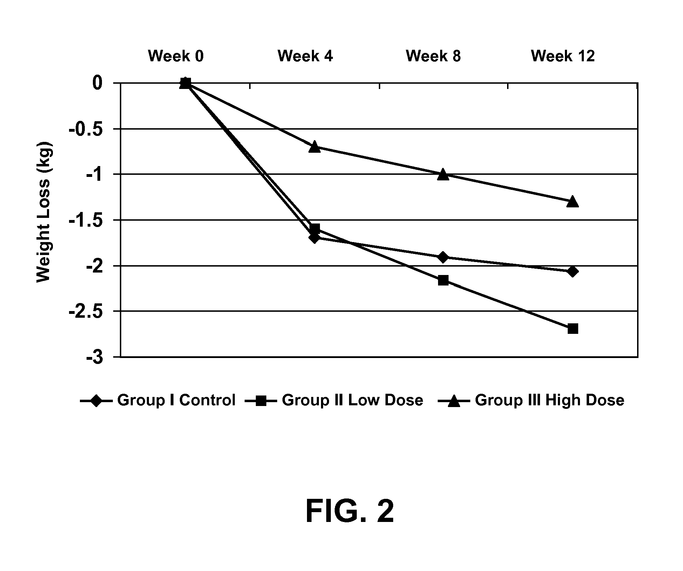

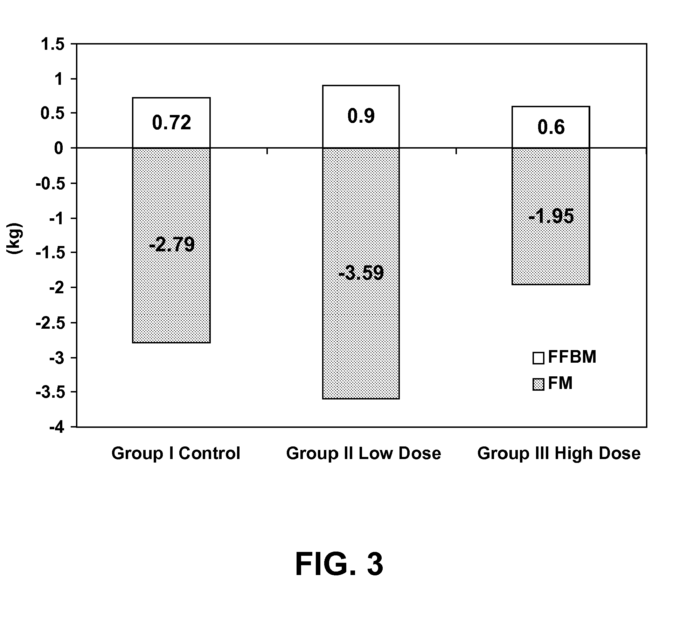

In accordance with Figure 1 and 2, after 12 weeks of study, weight reduction in patients was an average of 2.07 kg in Control Group I, 2.69 kg in Low Dose Group II, and 1.35 kg in High Dose Group III. From a standpoint of body composition (see Figure 3), on average in the Control Group I an increase is observed from 0.72 kg of Fat Free Body Mass (FFBM) and a decrease of 2.79 kg of fat mass (FM), in Low Dose Group II an increase of 0.90 kg of FFBM and a decrease of 3.59 kg of MG, whereas in High Dose Group III an increase of 0.60 kg of FFBM and a decrease of 1.95 kg of FM. On this basis there is a difference between the groups obtaining the greatest benefits of weight loss in Low Dose Group II.

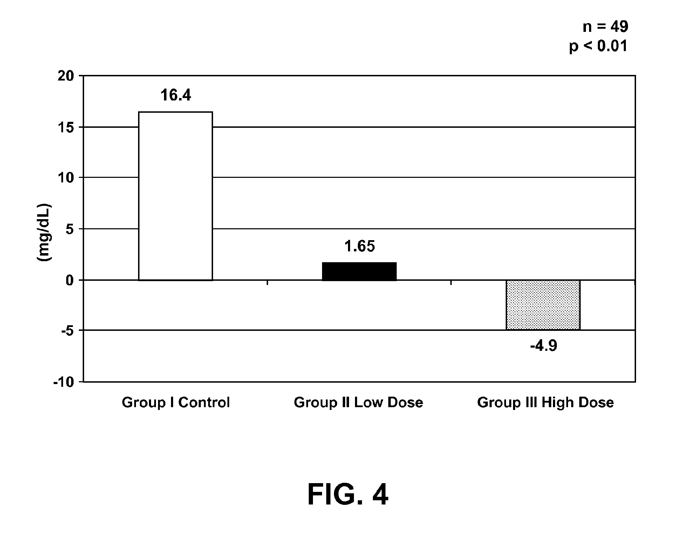

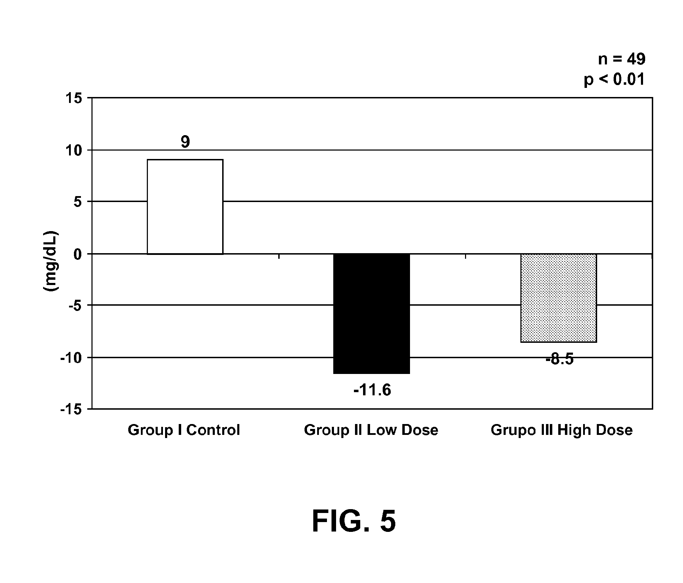

Simultaneously, there was an average increase in LDL cholesterol in plasma of 16.40 mg/dL in Control Group I and 1.65 mg/dL in Low Dose Group II, while the High Dose Group III showed a decrease of 4.9 mg/dL (see Figure 4). With respect to total cholesterol in plasma on average an increase of 9 mg/dL in Control Group I and a decrease of 11.6 mg/dL in the Low Dose Group II and of 8.5 mg/dL in the High Dose Group III (see Figure 5).

Based on the embodiments described above, it is contemplated that modifications of the environments of the embodiments described, as well the environments of the alternative embodiments will be considered obvious to a person skilled in the art of the art under this description. Therefore, it is considered that the claims cover those modifications and alternatives that are within the scope of the present invention or its equivalents.

## Claims

1. A composition for promoting control of total cholesterol and low density lipoprotein (LDL) in individuals, said composition is **characterized by** comprising:
green tea extract in a quantity of about 200 mg to about 300 mg;
guarana extract in a quantity of about 160 mg to about 260 mg;
yerba mate extract in quantity of about 100 mg to about 300 mg; and
conjugated linoleic acid in a quantity of about 700 mg to about 3400 mg.

2. The composition of claim 1, **characterized in that** said green tea extract contains a quantity of about 10 mg to about 15 mg of caffeine.

3. The composition of claim 1, **characterized in that** said guarana extract contains a quantity of about 35 mg to about 58 mg of caffeine.

4. The composition of claim 1, **characterized in that** said yerba mate extract contains a quantity of about 9 mg to about 27 mg of caffeine.

5. The composition of claim 1, **characterized in that** further contains a quantity of at least about 80 mg of epigallocatechin gallate (EGCG).

6. The composition of claim 1, **characterized in that** said green tea extract is standardized with a quantity of at least about 50% of epigallocatechin gallate (EGCG).

7. The composition of claim 1, **characterized in that** said conjugated linoleic acid is standardized with a quantity of at least about 50% of trans10, cis 12-CLA (10-12 CLA) and a quantify of at least about 50% of cis9, trans11-CLA (9-11 CLA).

8. The composition of claim 1, **characterized in that** further comprises an ingredient for satiating the appetite selected from a group consisting of glucomannan gum, xanthan gum, guar gum, and combinations thereof.

9. A composition for promoting weight loss in individuals, said composition is **characterized by** comprising:
green tea extract in a quantity of about 200 mg to about 300 mg;
guarana extract in a quantity of about 160 mg to about 260 mg;
yerba mate extract in quantity of about 100 mg to about 300 mg; and
conjugated linoleic acid in a quantity of about 700 mg to about 3400 mg.

10. The composition of claim 9, **characterized in that** said green tea extract contains a quantity of about 10 mg to about 15 mg of caffeine.

11. The composition of claim 9, **characterized in that** said guarana extract contains a quantity of about 35 mg to about 58 mg of caffeine.

12. The composition of claim 9, **characterized in that** said yerba mate extract contains a quantity of about 9 mg to about 27 mg of caffeine.

13. The composition of claim 9, **characterized in that** further contains a quantity of at least about 80 mg of epigallocatechin gallate (EGCG).

14. the composition of claim 9, **characterized in that** said green tea extract is standardized with a quantity of at least about 50% of epigallocatechin gallate (EGCG).

15. the composition of claim 9, **characterized in that** said conjugated linoleic acid is standardized with a quantity of at least about 50% of trans10, cis 12-CLA (10-12 CLA) and a quantify of at least about 50% of cis9, trans11-CLA (9-11 CLA).

16. the composition of claim 9, **characterized in that** further comprises an ingredient for satiating the appetite selected from a group consisting of glucomannan gum, xanthan gum, guar gum, and combinations thereof.

17. A composition for promoting thermogenesis in individuals, said composition is **characterized by** comprising:
green tea extract in a quantity of about 200 mg to about 300 mg;
guarana extract in a quantity of about 160 mg to about 260 mg;
yerba mate extract in quantity of about 100 mg to about 300 mg; and
conjugated linoleic acid in a quantity of about 700 mg to about 3400 mg.

18. The composition of claim 17, **characterized in that** said green tea extract contains a quantity of about 10 mg to about 15 mg of caffeine.

19. The composition of claim 17, **characterized in that** said guarana extract contains a quantity of about 35 mg to about 58 mg of caffeine.

20. The composition of claim 17, **characterized in that** said yerba mate extract contains a quantity of about 9 mg to about 27 mg of caffeine.

21. she composition of claim 17, **characterized in that** further contains a quantity of at least about 80 mg of epigallocatechin gallate (EGCG).

22. The composition of claim 17, **characterized in that** said green tea extract is standardized with a quantity of at least about 50% of epigallocatechin gallate (EGCG).

23. The composition of claim 17, **characterized in that** said conjugated linoleic acid is standardized with a quantity of at least about 50% of trans10, cis 12-CLA (10-12 CLA) and a quantify of at least about 50% of cis9, trans11-CLA (9-11 CLA).

24. The composition of claim 17, **characterized in that** further comprises an ingredient for satiating the appetite selected from a group consisting of glucomannan gum, xanthan gum, guar gum, and combinations thereof.

25. Use of a composition containing green tea extract in a quantity of about 200 mg to about 300 mg, guarana extract in a quantity of about 160 mg to about 260 mg, yerba mate extract in a quantity of about 100 mg to about 300 mg, and conjugated linoleic acid in a quantity of about 700 mg to about 3400 mg, for preparing a food product to promote control of total cholesterol and low density lipoprotein (LDL), and weight loss and/or thermogenesis in individuals.

26. The use of claim 25, **characterized in that** said green tea extract contains a quantity of about 10 mg to about 15 mg of caffeine.

27. The use of claim 25, **characterized in that** said guarana extract contains a quantity of about 35 mg to about 58 mg of caffeine.

28. The use of claim 25, **characterized in that** said yerba mate extract contains a quantity of about 9 mg to about 27 mg of caffeine.

29. The use of claim 25, **characterized in that** said composition further contains a quantity of at least about 80 mg of epigallocatechin gallate (EGCG).

30. The use of claim 25, **characterized in that** said green tea extract is standardized with a quantity of at least about 50% of epigallocatechin gallate (EGCG).

31. the uses of claim 25, **characterized in that** said conjugated linoleic acid is standardized with a quantity of at least about 50% of trans10, cis 12-CLA (10-12 CLA) and a quantify of at least about 50% of cis9, trans11-CLA (9-11 CLA).

32. The use of claim 25, **characterized in that** said composition further comprises an ingredient for satiating the appetite selected from a group consisting of glucomannan gum, xanthan gum, guar gum, and combinations thereof.

33. The use of claim 25, **characterized in that** said food product is selected from a group consisting of dairy products, juices and drinks with a pH lower than 4.5.

34. A method for treating control of total cholesterol and low density lipoprotein (LDL), weight loss and/or thermogenesis in individuals, said method is **characterized by** comprising the steps of:
administering a composition containing green tea extract in a quantity of about 200 mg to about 300 mg, guarana extract in a quantity of about 160 mg to about 260 mg, yerba mate extract in a quantity of about 100 mg to about 300 mg, and conjugated linoleic acid in a quantity of about 700 mg to about 3400 mg.

35. The method of claim 34, **characterized in that** said green tea extract contains a quantity of about 10 mg to about 15 mg of caffeine.

36. The method of claim 34, **characterized in that** said guarana extract contains a quantity of about 35 mg to about 58 mg of caffeine.

37. The method of claim 34, **characterized in that** said yerba mate extract contains a quantity of about 9 mg to about 27 mg of caffeine.

38. The method of claim 34, **characterized in that** said composition further contains a quantity of at least about 80 mg of epigallocatechin gallate (EGCG).

39. The method of claim 34, **characterized in that** said green tea extract is standardized with a quantity of at least about 50% of epigallocatechin gallate (EGCG).

40. The method of claim 34, **characterized in that** said conjugated linoleic acid is standardized with a quantity of at least about 50% of trans10, cis 12-CLA (10-12 CLA) and a quantify of at least about 50% of cis9, trans11-CLA (9-11 CLA).

41. The method of claim 34, **characterized in that** said composition further comprises an ingredient for satiating the appetite selected from a group consisting of glucomannan gum, xanthan gum, guar gum, and combinations thereof.
